# EUROPÄISCHE PATENTSCHRIFT

(11) **EP 1 206 292 B1**
(45) Veröffentlichungstag und Bekanntmachung des Hinweises auf die Patenterteilung: **14.01.2004**
(21) Anmeldenummer: 00964045.9
(22) Anmeldetag: 28.08.2000
(51) Int. Cl.: A61L 27/12

(54) **RESORBIERBARES KNOCHENERSATZ- UND KNOCHENAUFBAUMATERIAL**
RESORBABLE BONE REPLACEMENT AND BONE FORMATION MATERIAL
MATIERE RESORBABLE DE REMPLACEMENT OSSEUX ET DE FORMATION OSSEUSE

(30) Priorität: 26.08.1999 DE 19940717
(43) Veröffentlichungstag der Anmeldung: 22.05.2002
(73) Patentinhaber: Gerontocare GmbH, 64354 Reinheim (DE)
(72) Erfinder: HEIDE, Helmut, 64354 Reinheim (DE); PABST, Joachim, 64354 Reinheim (DE); DINKELAKER, Albrecht, 64354 Reinheim (DE); POBANTZ, Olav, 64354 Reinheim (DE)
(74) Vertreter: Boeters, Hans Dietrich, Dr.
(86) Internationale Anmeldenummer: PCT/EP2000/008382
(87) Internationale Veröffentlichungsnummer: WO 2001/013970

(56) Entgegenhaltungen:
- DE-A- 3 123 460
- DE-A- 3 810 803
- US-A- 4 195 366

## Beschreibung

### a) Phasenreinheit

Calciumorthophosphate, insbesondere das α- und β-Tricalciumphosphat (TCP) sowie der Hydroxylapatit (HAP) werden seit den 60iger Jahren als sog. bioaktive und resorbierbare Knochenersatzwerkstoffe erprobt und eingesetzt. Hierüber existiert eine umfangreiche werkstofftechnische und biomedizinische Fachliteratur, für die hier beispielhaft auf die ausführliche Zusammenstellung von K. deGroot verwiesen sei: Bioceramics of Calciumphosphates. K.deGroot (Editor) CRC Press, Bota Raton, FI. 1983, 1.
Die gute Bioverträglichkeit dieser Materialgruppe wird angesichts der weitgehenden chemischen Ähnlichkeit dieser Materialien zum anorganischen Bestandteil des Knochens, dem Hydroxylapatit verständlich. Die wissenschaftlichen Erkenntnisse der Pionierzeit der Forschung über diese Materialgruppe wird durch die oben zitierten Arbeit hinlänglich beschrieben:
Übereinstimmend wird über die gute Knochenverträglichkeit, die mehr oder weniger ausgeprägte Resorbierbarkeit und die sog. "Bioaktivität" berichtet, worunter man die positive chemische Wechselwirkung dieser Calciumphosphate mit dem lebenden Knochen versteht, die sich in einer direkten, bindegewebslosen Verbundbildung mit dem Knochen äussert.
Bis heute sind die exakten Korrelationen zwischen den werkstofflichen und biologischen Eigenschaften dieser Materialien noch vielfältig ungeklärt, und erst in letzter Zeit gewinnt man Erkenntnisse über die Zuordnung der werkstofflichen, thermodynamischen und kristallographischen Eigenarten dieser Materialien zu den biologischen Reaktionen des Knochens.

Man geht bei Fremdkörperreaktionen des Knochens im allgemeinen davon aus, daß Partikel mit einer Korngröße kleiner 20 Mikrometer von Makrophagen (Freßzellen) aufgenommen und abtransportiert bzw. verstoffwechselt werden (Phagozytose). Diese Vorgänge sind u.a. in den folgenden Veröffentlichungen angesprochen: Meachim et al.
In Biomaterials, 3 (4) (1982) 213-219 und Sioholm et al. In J. Pharmacol. Exp. Ther., 211 (3) (1979) 656-662.

In einer weiteren Arbeit berichtet deGroot (DeGroot et.al.: Die klinische Anwendbarkeit von Calciumphosphat Keramiken. Z.M.Fortbildung 75, 1985, 1938 - 1940) über den partikulären Zerfall von TCP in phagozytierbare Subpartikel, welche ins Lymphsystem gelangen können. Nach der Erfindung zugrundeliegenden Erkenntnissen haben diese Erscheinungen etwas mit dem Phasenbestand, der Phasenreinheit und dem Gefüge des damals untersuchten TCP-Materials zu tun. So besitzen nämlich die beiden wichtigsten TCP-Modifikationen, das α- und das β-TCP, trotz ihrer chemischen Analogie unterschiedliche Löslichkeiten und insbesondere unterschiedliche Umwandlungsverhalten im biologischen Milieu. In zahlreichen TCP-Materialien kommen die beiden Modifikationen des Tricalciumphosphates gemeinsam vor, wobei in aller Regel Phasen mit geringerer Stabilität (Gitterenergie und Löslichkeit) an den Korngrenzen angereichert werden. Bei fortschreitender chemischer Lösung und biologischen Abbauprozessen derartiger heterogener Materialien zerfällt ein solcher Werkstoff in der von De Groot beschriebenen Weise. Dieser Zerfallsmechanismus ist wegen der Anreicherung von "Fremdphasen" an den Korngrenzen der Hauptbestandteile des Materials selbst bei sehr geringen Phasenverunreinigungen wirksam. Hieraus folgt, daß derartige resorbierbare Implantatmaterialien sehr sorgfältig in phasenreiner Form synthetisiert werden müssen. Bei den dem Stande der Technik entsprechenden Materialien wird offensichtlich dieser Forderung nicht entsprochen. ( G. Bauer u. G. Hohnberger: Ursachen unterschiedlichen Verhaltens von bioaktiven Calcium Phosphatkeramiken im Organismus. cfi (Ber. d DKG) 66 (1989), 23-27)

### b) Porosität

### - Mikroporosität -

Unter Mikroporosität versteht man die mit bloßem Auge nicht mehr erkennbare Porosität eines keramischen Materials, d.h. Porenradien etwa kleiner gleich 20 Mikrometer (Römpp Chemie Lexikon, 7. Aufl. (1975), Franckh'sche Verlagshandlung), Stuttgart.

Neben der Phasenreinheit spielt die Art der Porenstruktur eines resorbierbaren Knochenersatzwerkstoffes eine wichtige Rolle.

Zunächst einmal ist festzustellen, daß die Erhöhung der Gefügeporosität die spezifische Oberfläche und damit auch die Resorbierbarkeit erhöht. Gleichzeitig erniedrigt sich die mechanische Festigkeit und die Neigung zum partikulären Zerfall nimmt zu.
Trotz dieses trivialen Zusammenhangs versucht man nach dem Stande der Technik eine möglichst hohe Resorptionsrate dadurch zu erreichen, daß man die innere Oberfläche des Materials durch Verwendung bzw. "Züchtung" möglichst feinpartikulärer Gefügebestandteile mit möglichst schwacher Korn/Korn-Bindung einstellt. Erwartungsgemäß sind daher Biomaterialien nach dem Stand der Technik, welche auf hohe Resorptionsraten "gezüchtet" werden, mechanisch so unvollkommen, daß sie im allg. nur für Anwendungen in Frage kommen, bei denen keine nennenswerten mechanischen Anforderungen gestellt werden. Die unkontrollierbare Zerlegung in mikroskopisch feine Subpartikel führt obendrein zur vermehrten Bildung von mehrkernigen Riesenzellen, was als ungünstige zelluläre Reaktion auf das betreffende Biomaterial angesehen werden muss.
Wünschenswert ist eine zügige, synchron mit der Restitution des neu gebildeten Knochen ablaufende Resorption des Implantates, ohne daß es dabei zu nennenswerten Zerlegungen des Gefüges kommt.

Nach dem Stande der Technik werden auch größere monolithische Formstücke aus derartigen mikroporösen Werkstoffen als Implantate eingesetzt, um größere Defekte des Knochens zu überbrücken. Dabei beobachtet man, daß bei solchen Aufbaumaterialien, bei denen lediglich mikroporöse Materialstrukturen vorliegen, nach oberflächlicher Resorption nach kurzer Zeit eine deutliche Stagnation der Resorptionsprozesse stattfindet und später sogar Abstoßungsprozesse auftreten können. Diese Erscheinungen sind nach der Erfindung zugrundeliegenden Erkenntnissen keineswegs auf die chemischen Materialeigenschaften der fraglichen Calciumphosphate zurückzuführen, sondern es liegt folgender negativer Effekt zu Grunde: Die Mikroporosität dieser Materialien besitzt nämlich eine kapillare Saugwirkung auf die Flüssigkeiten in der Implantatumgebung. Hierdurch werden diese Flüssigkeiten bis ins Innere der Implantatmaterialien eingesaugt und verbleiben dort über längere Zeiträume, während die Außenbezirke des Implantates von neugebildetem Knochen umwachsen werden. Knochenstrukturen und Blutgefäße vermögen die innen liegenden Bereiche nicht zu durchdringen und für einen diffusiven Stoffaustausch sind die Diffusionsstrecken zu groß. So kann es im Inneren solcher "unerreichbaren" Bezirke von monolithischen Implantatmaterialien zur Nekrose der zuvor durch die Kapillarität angesaugten Körperflüssigkeiten und Zellen kommen.

### -Makroporosität-

Nach Römpp Chemie Lexikon, 7. Aufl. (1975), Franckh'sche Verlagshandlung, Stuttgart, versteht man unter Makroporosität Porenradien größer gleich 20 Mikrometer.

Schon seit den 70er Jahren wurde die Möglichkeit untersucht, calciumphosphatische Implantatwerkstoffe mit einer offenen, durchgängigen Makroporosität einzusetzen. (K. Köster, H. Heide und R. König: Histologische Untersuchungen an der Grenzfläche zwischen Knochengewebe und Calciumphosphatkeramik usw., Z. Orthop. 115, (1977),693 - 699.), um dem Knochen die Möglichkeit einer schnellstmöglichen Penetration zu schaffen.

Dieser Gesichtspunkt spielt bei zahlreichen, dem Stande der Technik entsprechenden Produkten eine Rolle. Allerdings besitzen derartige, dem Stande der Technik entsprechende, makroporöse Produkte gravierende Nachteile, die nachstehend diskutiert werden sollen:
- Eine der gängigen Methoden zur Erzeugung einer makroporösen Struktur besteht in der Zugabe von Porosierungsmitteln, welche z.B. in Form von Schäumen oder kugelförmigen Kunststoffen eingebracht werden, die beim Erstarren einer hydraulisch abbindenden Ausgangsmasse bzw. beim keramischen Brand kugelförmige Poren entstehen läßt.
   Nachteilig bei dieser Porosierungsmethode ist, daß die Poren in der überwiegenden Zahl geschlossen sind. Sie stehen also für eine Penetration durch den einsprossenden Knochen nicht zur Verfügung und führen im Endeffekt nur zur Minderung der Festigkeit der Implantatregion.
- Einen ähnlichen Effekt besitzen Porosierungsverfahren, die auf mannigfache Weise durch Ausbrennen unregelmäßig geformter organischer "Spacer - Materialien" erzielt werden. Gängig sind in der keramischen Technologie z.B Sägespäne. Hierbei und bei zahlreichen ähnlichen Porosierungsmedien entstehen unregelmäßig verteilte Porenformen und -größen, die man als statistische Porosität bezeichnen kann. Sie werden in der keramischen Technologie zur Gewichtseinsparung der betreffenden Materialien und zur Verbesserung der Wärmedämmung eingesetzt. Auch bei Biomaterialien nach dem Stande der Technik werden in Anlehnung an diese Verfahren solche statistischen Porositäten benutzt. Für diesen Zweck sind sie allerdings aus folgenden Gründen gänzlich ungeeignet: Statistische Porositäten enthalten eine große Bandbreite der Porenradien-Verteilung und insbesondere auch zahlreiche geschlossene Poren sowie Porengänge mit "dead ends", die sich nicht für eine homogene und vollständige Penetration durch Knochen eignen.
- Aufbauend auf dieser Erkenntnis wird heute noch eine weitere Art von Porenstrukturen verwendet, die aus biogenen Produkten gewonnen wird. Es handelt sich hierbei entweder um einen sog. spongiösen Knochen z.B. aus Rinderknochen, der durch einen mehr oder weniger vollständigen Entzug der Eiweißbestandteile für diesen Zweck als Implantatmaterial konditioniert wird. Ferner nutzt man die porösen Strukturen von Korallen und bestimmten Algen um - unter Hinweis auf die biogene Entstehung - scheinbar optimale Porenstrukturen zu gewinnen. Abgesehen von den chemisch fragwürdigen Eigenschaften solcher Stoffe (z.B. undefinierte chemische Zusammensetzungen und immunulogische Probleme bei Rinderknochen usw. sowie völlig andere Chemismen, wie die des Knochens bei Verwendung von Algen und Korallen), so kann man auch diesen Porenstrukturen aus prinzipiellen Gründen keine besonders günstige Eignung zubilligen: Zunächst einmal kann festgestellt werden, daß die genannten Porenstrukturen als Endprodukte eines kybernetischen Anpassungsprozesses in den jeweiligen Ursprungsorganismen zwar optimal angepasste Systeme gebildet haben, die aber mit den biomechanischen Anforderungen im Implantatlager nichts mehr zu tun haben. (In einem solchen Biomaterial kann sich Knochen zwangsläufig nur in den offenen Porenräumen bilden, die in dem Ursprungsorganismus Löcher waren, d.h. eben nicht biofunktionelle Belastungszonen. Es kann also bestenfalles das "Negativ einer funktionellen Knochenstruktur entstehen.) Sieht man einmal von diesen mehr "philosophischen" Gründen ab, so sprechen gegen solche Strukturen auch die grundlegenden Argumente gegen "statistische Porositäten": Einwachsende Knochenstrukturen werden auch bei solchen biogenen Porenstrukturen z.B. durch zu kleine Poren behindert oder die einsprossenden Osteonen werden durch zahlreiche Richtungswechsel an einer möglichst schnellen biofunktionellen, lamellären Ausrichtung gehindert. Hierdurch wird die Bildung eines ungeregelt wachsenden "woven bones" geradezu provoziert.

Die Erfindung betrifft nun ein resorbierbares Knochenersatz- und Knochenaufbaumaterial (Augmentat-Werkstoff) auf Basis von porösem β-Tricalciumphosphat (β-TCP).

Bei dem erfindungsgemäßen Aufbaumaterial können die Makroporen für sich gesehen mit etwa 35 % zur Gesamtporosität, (d.h. Mikroporosität + Makroporosität) des Materials beitragen. Trotz der hohen Gesamtporosität von über 50 %, ist die Festigkeit dieses Implantatmateriales verglichen mit einer statistischen Porosität gleicher Größenordnung noch so hoch, daß Implantatformstücke immer noch sehr gut handhabar sind. Es handelt sich hierbei jedoch zweifellos um so geringe Festigkeiten, daß funktionelle Festigkeiten der Implantatstellen ohne zusätzliche mechanische Stützeinrichtungen, wie z. B. einen "fixateur externe" oder die bekannten Verschraubungen mit Platten usw. in vielen Fällen unmittelbar nach der Implantation unumgänglich sind. Einer der entscheidenden Vorteile des erfindungsgemäßen Aufbaumaterials ist jedoch, daß die Vernetzung der Implantatstrukur mit funktionell ausgerichteten und räumlich vernetzten Knochenstrukturen sehr zügig vonstatten geht, so daß im Vergleich mit anderen Implantatmaterialien, welche dem Stand der Technik entsprechen, eine sehr rasche Wiederherstellung der funktionellen Belastbarkeit der Implantatzone erreicht wird. Hierdurch wird die naturgesetzlich geringe Festigkeit calciumphosphatischer Werkstoffe allein durch die erfindungsgemäße Makroporenstruktur kompensiert. Bereits während der Penetrationsphase derartiger Strukturen sorgen die einsprossenden, in ihrer funktionellen Ausrichtung an die Belastungsverhältnisse bereits angepassten Osteonen für eine rasche Versorgung des gesamten Implantatbereiches mit Gefäßen und damit für eine zügige Resorption des erfindungsgemäßen Aufbaumaterials, wobei simultan sehr rasch ein biofunktionell belastbarer Zustand erreicht wird. Der allgemeinen Forderung nach einer schnellstmöglichen Restitution der Implantatstelle entspricht das erfindungsgemäße Knochenersatz- und Aufbaumaterial allein durch die beschriebenen Merkmale hinsichtlich
- der Phasenreinheit
- der Mikroporosität und
- der funktionsangepassten Makroporosität
in idealer Weise. Diese positiven Faktoren lassen sich noch steigern, indem die erfindungsgemäßen Implantatmaterialien mit den wachstumsfördernden Bestandteilen des Patientenblutes, dem sog. platelet rich plasma, bzw. sog, bone morphogenic proteins kombiniert werden. Dies kann z.B. dadurch geschehen, daß die mikro- und makroporösen Räume der Implantatmaterialien unmittelbar vor der Operation mit flüssig eingestellten Zubereitungen der wachstumsfördernden Medien getränkt werden.

Weitere Gesichtspunkte der Lösung der technischen Aufgaben und Vorteile des erfindungsgemäßen Knochenersatz- und Aufbaumaterials sind im folgenden zusammengestellt.

Die Erfindung betrifft ferner ein Aufbaumaterial, das dadurch gekennzeichnet ist, daß die chemische und kristalline Reinheit, der Gefügeaufbau, die Mikroporosität und die Makroporosität des Augmentat-Werkstoffs eine zügige, von Fremdkörperreaktionen freie, biochemisch orientierte Integration und Resorption im Knochen ermöglichen.

Ferner kann das erfindungsgemäße Aufbaumaterial dadurch gekennzeichnet sein, daß das Material zu mindestens 99,5 % aus reinem β-Tricalciumphosphat (β-TCP) besteht.

Ferner kann das erfindungsgemäße Aufbaumaterial dadurch herstellbar sein, daß man β-Tricalciumphosphats (β-TCP) mindestens 2-mal und insbesondere mindestens 3-mal brennt und die Entstehung der thermodynamisch stabilen Nachbarphasen des β-TCP verhindert.

Ferner kann das erfindungsgemäße Aufbaumaterial dadurch herstellbar sein, daß man
(i) ein Phosphatpulver einer chemischen Zusammensetzung brennt, deren Brennrückstand theoretisch chemisch reines Tricalciumphosphat als zusammengesintertes Vorsynthese-Produkt ergibt, und dieses Vorsynthese-Produkt pulverisiert,
(ii) gegebenenfalls das pulverisierte Vorsynthese-Produkt zusammen mit Phosphatpulver gemäß Stufe (i) brennt und das anfallende Material pulverisiert und gegebenenfalls Stufe (ii) einmal oder mehrmals wiederholt,
(iii) das bei Stufe (i) oder Stufe (ii) anfallende pulverisierte Produkt zusammen mit Phospatpulver gemäß Stufe (i) zu Rohlingen preßt und die gebildeten Rohlinge einem keramischen Endbrand unterwirft und
(iv) die gepreßten oder gebrannten Rohlinge mit Röhrenporen versieht.

Ferner kann das erfindungsgemäße Aufbaumaterial dadurch herstellbar sein, daß man
(i) von einem Vorsynthese-Produkt ausgeht, das dadurch erhältlich ist, daß man ein Phosphatpulver einer chemischen Zusammensetzung brennt, deren Brennrückstand theoretisch chemisch reines Tricalciumphosphat als zusammengesintertes Vorsynthese-Produkt ergibt und dieses Vorsynthese-Produkt pulverisiert,
(ii) gegebenenfalls das pulverisierte Vorsynthese-Produkt zusammen mit Phosphatpulver gemäß Stufe (i) brennt und das anfallende Material pulverisiert und gegebenenfalls Stufe (ii) einmal oder mehrmals wiederholt,
(iii) das bei Stufe (i) oder Stufe (ii) anfallende pulverisierte Produkt zusammen mit Phospatpulver gemäß Stufe (i) zu Rohlingen preßt und die gebildeten Rohlinge einem keramischen Endbrand unterwirft und
(iv) die gepreßten oder gebrannten Rohlinge mit Röhrenporen versieht.

Ferner kann das erfindungsgemäße Aufbaumaterial dadurch erhältlich sein, daß man bei einer Temperatur unterhalb 1200 °C im Zustandsgebiet von β-Tricalciumphosphat (β-TCP) brennt.

Ferner kann das erfindungsgemäße Aufbaumaterial dadurch erhältlich sein, daß man bei Stufe (ii) und/oder Stufe (iii) 1 bis 50 Gew.-%, insbesondere 1 bis 25 Gew.-% Phosphatpulver einsetzt (bezogen auf das Gesamtgewicht von Phosphatpulver und bereits gebranntem Material).

Ferner kann das erfindungsgemäße Aufbaumaterial dadurch gekennzeichnet sein, daß das Sintergefüge eine einheitliche, durchgängige Mikroporosität mit Porenweiten im Bereich von 2 bis 15 µm und insbesondere 4 bis 10 µm aufweist und/oder die Matrix des Augmentat-Werkstoffs bis zur Mikroporosität dichtgesintert ist, insbesondere im Sintergefüge lose gebundene und/oder phagozytierbare Mikropartikel mit einem Durchmesser von höchstens 15 µm fehlen.

Sehr günstige zelluläre Reaktionen beobachtet man nun, wenn das Knochenersatzmaterial über die erfindungsgemäßen Gefügeparameter verfügt: Durch den später zu diskutierenden Herstellungsgang zeichnet sich das erfindungsgemäße Material durch eine offene, interkonnektierende Mikroporosität mit Porenweiten zwischen 2 bis 15 µm aus. Die keramische Matrix selbst stellt ein Netzwerk von dichten fest miteinander versinterten Strukturelementen dar, bei dem lose eingebundene Subpartikel fehlen, die durch die Zellaktivitäten herausgelöst werden könnten.

Ferner kann das erfindungsgemäße Aufbaumaterial durch eine Mikroporosität von 20 Vol.-% oder mehr, vorzugsweise von 20 bis 40 Vol.-%, und insbesondere 30 Vol.-% oder mehr der Gesamtporosität (aus Mikro- und Makroporosität) gekennzeichnet sein.

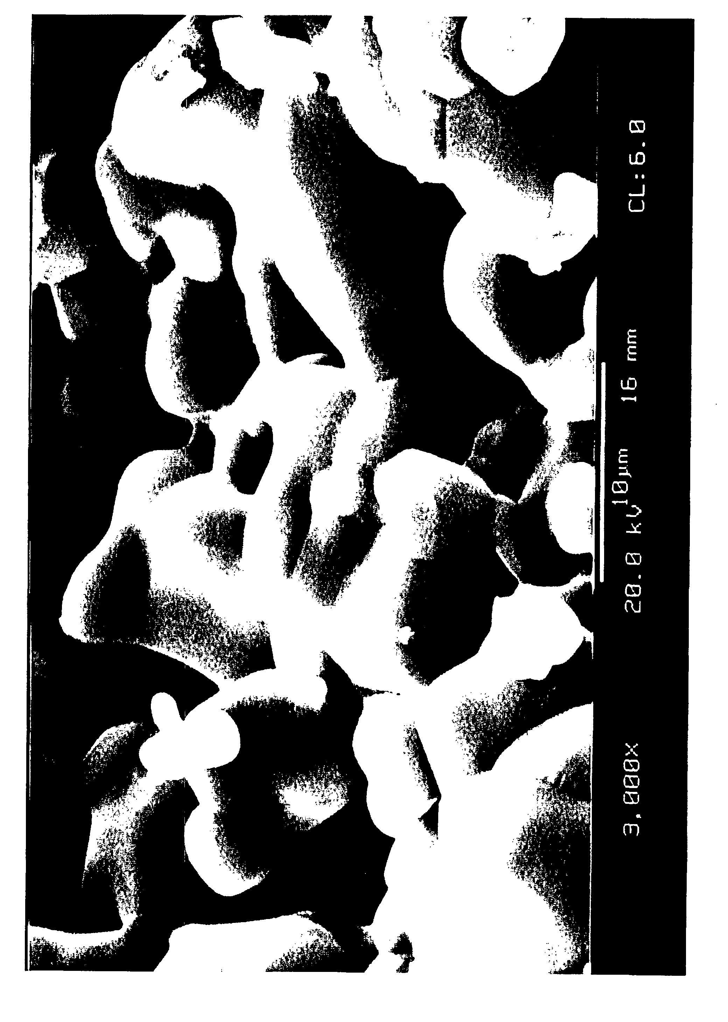

Charakteristisch bei dem erfindungsgemäßen Mikrogefüge sind ausserdem die abgerundeten Oberflächen der gefügebildenden Strukturelemente (vgl. Fig. 1), die sich gegenüber den lebenden Zellen im Implantatlager besonders günstig verhalten, da hierdurch mechanisch induzierte Reizungen des Lagergewebes weitgehend vermieden werden. Diese abgerundeten Gefügeelemente bewirken ausserdem eine Stress- und Spannungsminimierung im werkstofftechnischen Sinne, so daß die erfindungsgemäßen Werkstoffe trotz ihrer verhältnismäßig hohen Mikroporosität von mehr als 30 Vol.% ein Optimum an mechanischer Festigkeit aufweisen.

Ferner kann das erfindungsgemäße Aufbaumaterial dadurch erhältlich sein, daß man den gepreßten Rohling mit Hilfe einer gegebenenfalls mehrteiligen Preßform mit Röhrenporen versieht.

Ferner kann das erfindungsgemäße Aufbaumaterial dadurch erhältlich sein, daß man den gebrannten Rohling durch Fräsen oder Bohren mit Röhrenporen versieht.

Ferner kann das erfindungsgemäße Aufbaumaterial dadurch gekennzeichnet sein, daß das Aufbaumaterial in Blockform vorliegt, wobei jeder Block mit 2- oder 3-dimensional orientierten makroskopischen Röhrenporen durchzogen ist, die jeweils senkrecht zur Blockoberfläche oder einer gedachten und durch den Block gelegten oder an den Block angelegten Ebene stehen und ein interkonnektierendes System aus Röhrenporen bilden.

Ferner kann das erfindungsgemäße Aufbaumaterial dadurch gekennzeichnet sein, daß sich ein für eine Implantation vorgesehener Block mit seinen Röhrenporen für eine Implantation oder bei einer der Implantation vorhergehenden Bearbeitung derart ausrichten läßt, daß wenigstens eine Orientierungsrichtung der Röhrenporen einer biomechanisch oder biofunktionell vorgesehenen Wachstumsrichtung entspricht.

Ferner kann das erfindungsgemäße Aufbaumaterial durch Röhrenporen mit Radien im Bereich von 100 bis 2000 µm und insbesondere 500 bis 2000 µm gekennzeichnet sein.

Im Gegensatz zum Stand der Technik ist das erfindungsgemäße Knochenersatz- und Aufbaumaterial mit einer sehr regelmäßig ausgerichteten Röhrenporosität ausgestattet, welche mit Radien vorzugsweise zwischen 500 und 2000 µm optimale Größenverhältnisse für das Einsprossen von Osteonen aufweist. Derartige parallel angeordnete Poren durchziehen die erfindungsgemäßen Materialien in mindestens zwei, in besonderen Anwendungsfällen sogar drei senkrecht aufeinander stehenden Röhrensystemen. Zur optimalen Anpassung an die funktionelle Aufgabe sollte eine der Röhrenausrichtungen bei der Implantation mit der Hauptwachstumsrichtung des randständigen Wirtsknoches übereinstimmen. Da die senkrecht aufeinander stehenden Porensysteme der erfindungsgemäßen Implantatmaterialien in allen Ebenen interkonnektieren, vernetzen sich die einsprossenden Knochenstrukturen sehr rasch zu einem gut vaskularisierten räumlichen Netz von tragfähigen Knochenstrukturen. Hierdurch stellt das erfindungsgemäße Knochenaufbaumaterial im wahrsten Sinne des Wortes ein optimales Leitschienensystem dar.

Damit stimmen überein Untersuchungen von Klawitter et al. (Klawitter,J.J. et al.: An Evaluation of Bone Growth into Porous High Density PE. J.Biomed.Res.10:311, 1976) wonach die kleinsten funktionellen Bauelemente des Knochens, die Osteonen, d.s. schlauchartige Strukturen mit kompletten Versorgungsorganen für die Erhaltung der Lebensfunktionen, nur durch Porengänge einwachsen können, die wenigstens 100 µm Porenweite aufweisen. Kleinere Porensysteme lassen eine biofunktionelle Penetration mit lebendem Knochen nicht zu. Hieraus folgt, daß Biomaterialien mit statistischen Porensystemen, wie sie dem heutigen Stande der Technik entsprechen, keine befriedigende Lösung sein können.

Ferner kann das erfindungsgemäße Aufbaumaterial dadurch gekennzeichnet sein, daß die Röhrenporen das in Blockform vorliegende Aufbaumaterial in einem definierten gegenseitigen Abstand durchdringen, insbesondere in einem Abstand, der einer Wandstärke von nicht mehr als 1500 bis 4000 µm und insbesondere 2000 bis 3000 µm entspricht.

Nach weiteren der Erfindung zugrundeliegenden Untersuchungen liegen bei monolithischen Materialstrukturen, die lediglich über eine Mikroporosität verfügen, die kritischen Materialstärken oberhalb 3 - 4 mm. Ist die Wandstärke geringer, so können die Körperflüssigkeiten durch diffusive Prozesse mit dem lebenden Umgebungsgewebe ausgetauscht werden, so daß keine nekrotischen Prozesse stattfinden.

Die zuvor im Abschnitt "Mikroporosität" abgeleitete Forderung nach Wandstärken von höchstens 3 bis 4 mm wird in dem erfindungsgemäßen, definiert makroporösen Material dadurch erfüllt, daß die Röhrenporen so eng angelegt sind, daß die Materialstärken an keiner Stelle größer als etwa 3 mm sind.

Ferner kann das erfindungsgemäße Aufbaumaterial durch eine Gesamtporosität (aus Mikro- und Makroporosität) von mehr als 50 Vol.-% gekennzeichnet sein kann.

Ferner kann das erfindungsgemäße Aufbaumaterial durch eine Makroporosität von 25 bis 50 Vol.-% und insbesondere 30 bis 40 Vol.-% der Gesamtporosität (aus Mikro- und Makroporosität) gekennzeichnet sein.

Ferner kann das erfindungsgemäße Aufbaumaterial dadurch gekennzeichnet sein, daß es sich bei der Blockform um eine einfache geometrische Form handelt, insbesondere die eines Würfels, Quaders, Kegels, Konus oder einer Scheibe.

Ferner kann das erfindungsgemäße Aufbaumaterial dadurch gekennzeichnet sein, daß es sich um Halbzeug handelt, insbesondere zur mechanischen Nachbearbeitung, vorzugsweise für eine individuelle Anpassung bei einem Knochendefekt in der Mund- oder Kieferheilkunde, der orthopädischen Chirurgie oder der Unfallchirurgie.

Ferner kann das erfindungsgemäße Aufbaumaterial dadurch gekennzeichnet sein, daß das Material nur bis zu einem Grad verdichtet ist, insbesondere gebrannt oder gesintert ist, daß es mit dem Arzt zur Verfügung stehenden Werkzeugen bearbeitet werden kann, insbesondere mit einer Raspel, einer Feile, einem Skalpell oder einem zahnärztlichen Instrument.

Ferner kann das erfindungsgemäße Aufbaumaterial dadurch gekennzeichnet sein, daß es mit Hilfe eines medizinischen CAD/CAM-Verfahrens in die Form einer Individualprothese gebracht worden ist.

Nachstehend wird die Erfindung durch Figuren und Ausführungsbeispiele näher erläutert. Es zeigen:
Fig. 1 das Mikrogefüge eines erfindungsgemäßen Augmentat-Werkstoffs,
Fig. 2a bis 2e Beispiele für erfindungsgemäße Augmentate als Halbzeuge,
Fig. 3a ein erfindungsgemäßes Alveolar-Augmentat,
Fig. 3b ein erfindungsgemäßes Augmentat für einen Trepanationsverschluß und
Fig. 3c ein erfindungsgemäßes Augmentat als Sinuslift.

### Ausführungsbeispiele

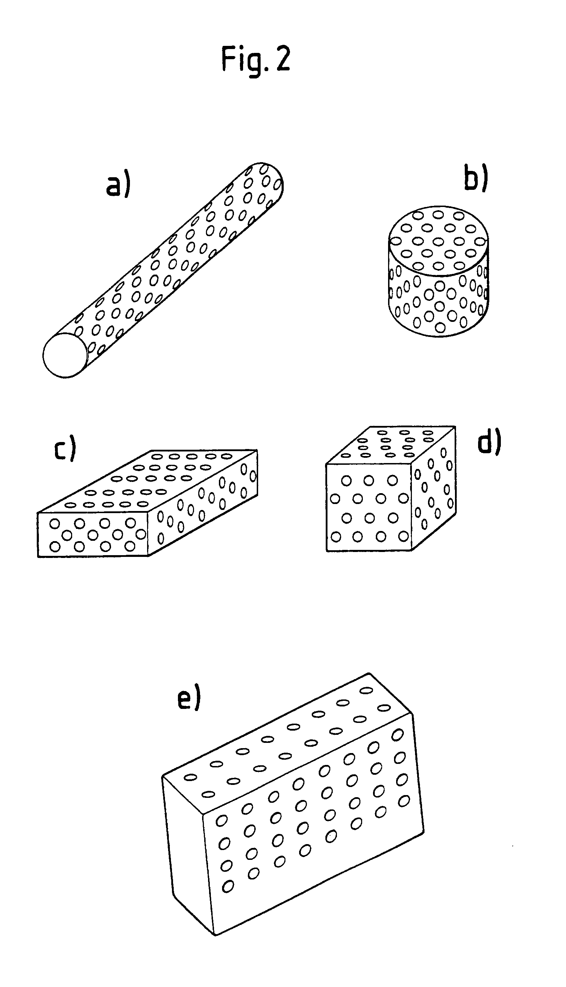

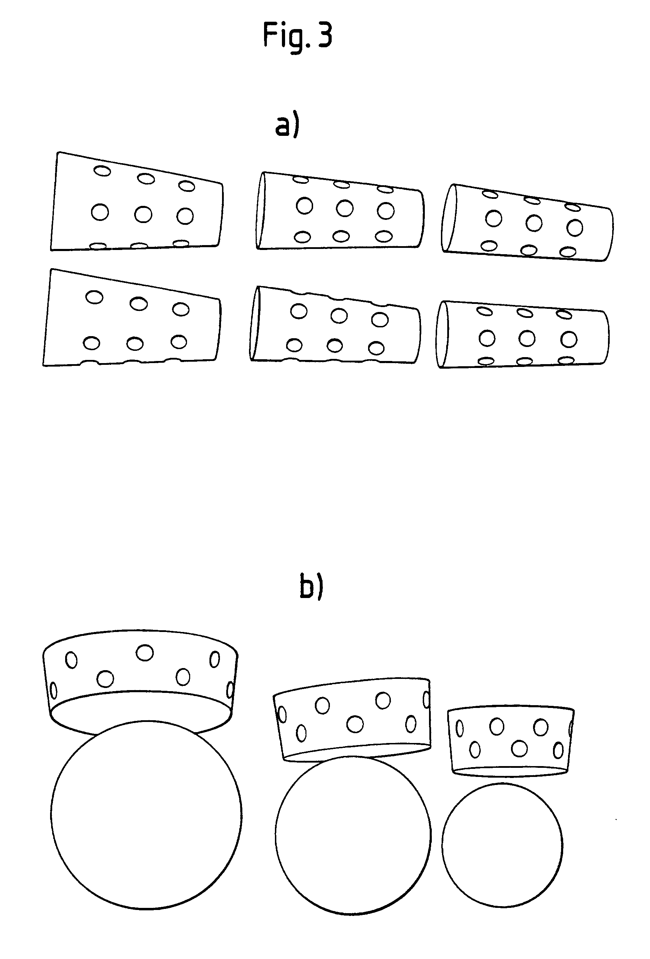

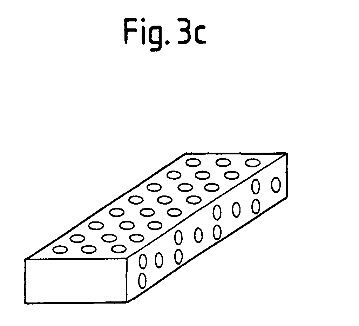

1. Nach einer vorteilhaften Ausführungsart läßt sich das erfindungsgemäße keramische Implantatmaterial aus den beiden anorganischen Materialien Calciumhydrogenphosphat und Calciumcarbonat aus stöchiometrischen Mischungen durch Sinterung gemäß der Formel 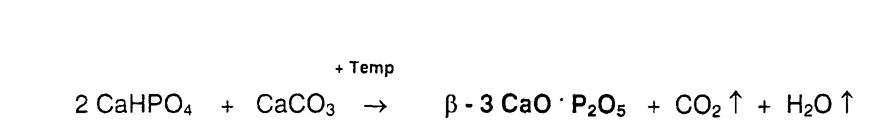 synthetisieren.
   Die Synthese erfolgt bei Temperaturen unterhalb 1200°C im Zustandsgebiet des β - TCP . Zur Vermeidung der Entstehung unerwünschter Nebenphasen wie die des α-TCP, von amorphen Phasen sowie von Hydroxylapatit erfolgt die Rektifizierung des Materials durch mehrfaches Sintern im o.a. Temperaturbereich.
2. Die Formgebung des erfindungsgemäßen Implantatmaterials erfolgt nach einer vorteilhaften Weise, indem man aus dem phasenreinen TCP-Pulver lange zylindrische Rohlinge presst, diese bei T< 1200°C brennt und die so gewonnenen Rohlinge mittels spanabhebender Verfahren (Fräsen, Drehen und Bohren) in die gewünschte Form bringt
3. Die Erzeugung handhabbarer Implantate aus dem erfindungsgemäßen Knochenersatzmaterial kann nach einer vorteilhaften Ausführungsart in Form von stangenartigen zylindischen, quaderförmigen, würfelförmigen sowie anderen Halbzeugen hergestellt werden, welche vom Operateur mit geeigneten Werkzeugen (Feilen, Raspeln Sägen usw.) in die gewünschte Form gebracht werden können (vgl. Fig. 2) Diese Halbzeuge können durch übliche Presstechniken aus Pulvern, aber auch durch Gießtechniken, wie sie in der Keramik üblich sind gefertigt werden. Die Erzeugung der erfindungsgemäßen Röhrenporosität wird nach dem keramischen Brand dieser Formteile durch Bohren und Fräsen erzeugt.
   Sie kann aber auch durch Pulverpressen in mehrteiligen Pressformen erfolgen
4. Spezielle Implantate aus dem erfindungsgemäßen Material, welche in bestimmten Anwendungsfeldern eingesetzt werden, können in großer Stückzahl in standardisierten Größenabstufungen aus Rohlingen durch z.B. spanabhebende Methoden gefertigt werden. Einige vorteilhafte Ausführungsarten sind in den **Fig. 3a -c** dargestellt. Z.B. zeigt die **Fig. 3a** ein sog. Alveolar-Augmentat für die Auffüllung einer Alveole nach Extraktion eines Zahnes, **Fig. 3b** eine konische Scheibe, die zum Verschluß einer Trepanationsöffnung des Schädeldaches dient, **Fig. 3c** zeigt eine vorteilhafte Ausführungsart eines flachen Augmentates zur Unterfütterung b.z.w. Anhebung eines atrophierten Kieferkamms, sog. Sinuslift.
5. Es wurden 2 Mol Calciumhydrogenphosphat und 1 Mol Calciumcarbonat als Pulver gemischt, zu einem Formkörper verpreßt, in einen Keramiktiegel überführt und bei 1100 °C 24 Stunden gesintert. Der Sinterkörper wurde gebrochen und gemahlen, mit 1 % einer unreagierten pulverförmigen Mischung der oben angegebenen Rezeptur versetzt und mit ihr innig vermischt. Danach wurde das Gemisch zu einem Formkörper verpreßt und bei 1100 °C nochmals 24 Stunden gesintert. Nach dem Abkühlen wurde der erhaltene Sinterkörper mechanisch bearbeitet und in eine Form gemäß Fig. 2e gebracht. Das Formteil wurde abschließend nochmals 24 Stunden bei 950°C gebrannt.

## Patentansprüche

1. Resorbierbares Knochenersatz- und Knochenaufbaumaterial (Augmentat-Werkstoff) auf Basis von porösem β-Tricalciumphosphat (β-TCP), dadurch **herstellbar,** daß man
(a) ein Phosphatpulver einer chemischen Zusammensetzung brennt, deren Brennrückstand theoretisch chemisch reines Tricalciumphosphat ergibt, und
(b) die gebrannten Rohlinge mit Röhrenporen versieht,
**dadurch gekennzeichnet,**
**daß** man β-Tricalciumphosphat (β-TCP) mindestens 2-mal und insbesondere mindestens 3-mal brennt und die Entstehung der thermodynamisch stabilen Nachbarphasen des β-TCP verhindert, indem man
(i) das gemäß Stufe (a) anfallende Vorsynthese-Produkt pulverisiert,
(ii) gegebenenfalls das pulverisierte Vorsynthese-Produkt zusammen mit Phosphatpulver gemäß Stufe (a) brennt und das anfallende Material pulverisiert und gegebenenfalls Stufe (ii) einmal oder mehrmals wiederholt,
(iii) das bei Stufe (i) oder Stufe (ii) anfallende pulverisierte Produkt zusammen mit Phosphatpulver gemäß Stufe (a) zu Rohlingen preßt und die gebildeten Rohlinge einem keramischen Endbrand unterwirft und
(iv) die gepreßten oder gebrannten Rohlinge, die zu mindestens 99,5 % aus reinem β-Tricalciumphosphat (β-TCP) bestehen, Stufe (b) unterwirft.

2. Aufbaumaterial nach Anspruch 1, **dadurch gekennzeichnet, daß** die chemische und kristalline Reinheit, der Gefügeaufbau, die Mikroporosität und die Makroporosität des Augmentat-Werkstoffs eine zügige, von Fremdkörperreaktionen freie, biochemisch orientierte Integration und Resorption im Knochen ermöglichen.

3. Aufbaumaterial nach einem der vorhergehenden Ansprüche, dadurch herstellbar, daß man
(i) von einem Vorsynthese-Produkt ausgeht, das dadurch **erhältlich** ist, daß man ein Phosphatpulver einer chemischen Zusammensetzung brennt, deren Brennrückstand theoretisch chemisch reines Tricalciumphosphat als Vorsynthese-Produkt ergibt und dieses Vorsynthese-Produkt pulverisiert,
(ii) gegebenenfalls das pulverisierte Vorsynthese-Produkt zusammen mit Phosphatpulver gemäß Stufe (i) brennt und das anfallende Material pulverisiert und gegebenenfalls Stufe (ii) einmal oder mehrmals wiederholt,
(iii) das bei Stufe (i) oder Stufe (ii) anfallende pulverisierte Produkt zusammen mit Phospatpulver gemäß Stufe (i) zu Rohlingen preßt und die gebildeten Rohlinge einem keramischen Endbrand unterwirft und
(iv) die gepreßten oder gebrannten Rohlinge mit Röhrenporen versieht.

4. Aufbaumaterial nach Anspruch 1 oder 3, dadurch erhältlich, daß man bei einer Temperatur unterhalb 1200 °C im Zustandsgebiet von β-Tricalciumphosphat (β-TCP) brennt.

5. Aufbaumaterial nach einem der Ansprüche 1,3 oder 5, dadurch erhältlich, daß man bei Stufe (ii) und/oder Stufe (iii) 1 bis 50 Gew.-%, insbesondere 1 bis 25 Gew.-% Phosphatpulver einsetzt (bezogen auf das Gesamtgewicht von Phosphatpulver und bereits gebranntem Material).

6. Aufbaumaterial nach einem der vorhergehenden Ansprüche, **dadurch gekennzeichnet, daß** die Matrix des Augmentat-Werkstoffs bis zur Mikroporosität dichtgesintert ist.

7. Aufbaumaterial nach einem der vorhergehenden Ansprüche, **gekennzeichnet durch** eine Mikroporosität von 20 Vol.-% oder mehr, vorzugsweise von 20 bis 40 Vol.-%, und insbesondere von 30 Vol.-% oder mehr, bezogen auf Gesamtporosität (aus Mikro- und Makroporosität).

8. Aufbaumaterial nach einem der vorhergehenden Ansprüche, dadurch **erhältlich,** daß man den gepreßten Rohling mit Hilfe einer gegebenenfalls mehrteiligen Preßform mit Röhrenporen versieht.

9. Aufbaumaterial nach einem der vorhergehenden Ansprüche, dadurch **erhältlich,** daß man den gebrannten Rohling durch Fräsen oder Bohren mit Röhrenporen versieht.

10. Aufbaumaterial nach einem der vorhergehenden Ansprüche, **dadurch gekennzeichnet, daß** das Aufbaumaterial in Blockform vorliegt, wobei jeder Block mit 2- oder 3-dimensional orientierten makroskopischen Röhrenporen durchzogen ist, die jeweils senkrecht zur Blockoberfläche oder einer gedachten und durch den Block gelegten oder an den Block angelegten Ebene stehen und ein interkonnektierendes System aus Röhrenporen bilden.

11. Aufbaumaterial nach Anspruch 10, **dadurch gekennzeichnet, daß** sich ein für eine Implantation vorgesehener Block mit seinen Röhrenporen für eine Implantation oder bei einer der Implantation vorhergehenden Bearbeitung derart ausrichten läßt, daß wenigstens eine Orientierungsrichtung der Röhrenporen einer biomechanisch oder biofunktionell vorgesehenen Wachstumsrichtung entspricht.

12. Aufbaumaterial nach einem der vorhergehenden Ansprüche, **gekennzeichnet durch** Röhrenporen mit Radien im Bereich von 100 bis 2000 µm und insbesondere 500 bis 2000 µm.

13. Aufbaumaterial nach einem der vorhergehenden Ansprüche, **dadurch gekennzeichnet, daß** die Röhrenporen das in Blockform vorliegende Aufbaumaterial in einem definierten gegenseitigen Abstand durchdringen, insbesondere in einem Abstand, der einer Wandstärke von nicht mehr als 1500 bis 4000 µm und insbesondere 2000 bis 3000 µm entspricht.

14. Aufbaumaterial nach einem der vorhergehenden Ansprüche, **gekennzeichnet durch** eine Gesamtporosität (aus Mikro- und Makroporosität) von mehr als 50 Vol.-%.

15. Aufbaumaterial nach einem der vorhergehenden Ansprüche, **gekennzeichnet durch** eine Makroporosität von 25 bis 50 Vol.-% und insbesondere 30 bis 40 Vol.-%, bezogen auf Gesamtporosität (aus Mikro- und Makroporosität).

16. Aufbaumaterial nach einem der vorhergehenden Ansprüche, **dadurch gekennzeichnet, daß** es sich bei der Blockform um eine einfache geometrische Form handelt, insbesondere die eines Würfels, Quaders, Kegels, Konus oder einer Scheibe.

17. Aufbaumaterial nach einem der vorhergehenden Ansprüche, **dadurch gekennzeichnet, daß** es sich um Halbzeug handelt, insbesondere zur mechanischen Nachbearbeitung, vorzugsweise für eine individuelle Anpassung bei einem Knochendefekt in der Mund- oder Kieferheilkunde, der orthopädischen Chirurgie oder der Unfallchirurgie.

18. Aufbaumaterial nach einem der Ansprüche 11 bis 17, **dadurch gekennzeichnet, daß** das Material nur bis zu einem Grad verdichtet ist, insbesondere gebrannt oder gesintert ist, daß es mit dem Arzt zur Verfügung stehenden Werkzeugen bearbeitet werden kann, insbesondere mit einer Raspel, einer Feile,
einem Skalpell oder einem zahnärztlichen Instrument.

19. Individualprothese, dadurch herstellbar, daß man ein Aufbaumaterial gemäß einem der Ansprüche 11 bis 17 mit Hilfe eines medizinischen CAD/CAM-Verfahrens in die Form der Individualprothese bringt.

## Claims

1. Resorbable bone replacement and bone formation material (augmentation material) based on porous β-tricalcium phosphate (β-TCP), which can be produced by
(a) baking a phosphate powder of a chemical composition the residue on baking of which yields theoretically chemically pure tricalcium phosphate and
(b) providing the baked planks with tubular pores,
**characterised in that**
β-tricalcium phosphate (β-TCP) is baked at least twice and especially at least three times and the formation of the thermodynamically stable adjacent phases of β-TCP is prevented by
(i) powdering the presynthesis product obtained according to step (a),
(ii) optionally baking the powedered presynthesis product together with phosphate powder according to step (a) and powdering the material obtained and optionally repeating step (ii) once or more than once,
(iii) compressing the powdered product obtained in step (i) or step (ii) together with phosphate powder according to step (a) to form blanks and subjecting the blanks formed to final ceramic baking and
(iv) subjecting the compressed or baked blanks, which consists to at least 99.5 % of pure β-tricalcium phosphate (β-TCP), to step (b).

2. Formation material according to claim 1, **characterised in that** the chemical and crystalline purity, the fabric structure, the microporosity and the macroporosity of the augmentation material make possible rapid, foreign-body-reaction-free, biochemically orientated integration and resorption in bone.

3. Formation material according to one of the preceding claims, which can be produced by
(i) starting from a presynthesis product, obtainable by baking a phosphate powder of a chemical composition the residue on baking of which yields theoretically chemically pure tricalcium phosphate as a presynthesis product, and powdering that presynthesis product,
(ii) optionally baking the powdered presynthesis product together with phosphate powder according to step (i) and powdering the material obtained and optionally repeating step (ii) once or more than once,
(iii) compressing the powdered product obtained in step (i) or step (ii) together with phosphate powder according to step (i) to form blanks and subjecting the blanks formed to final ceramic baking and
(iv) providing the compressed or baked blanks with tubular pores.

4. Formation material according to claim 1 or 3, obtainable by baking at a temperature below 1200°C in the β-tricalcium (β-TCP) phase region.

5. Formation material according to one of claims 1, 3 or 5, obtainable by using in step (ii) and/or step (iii) from 1 to 50 % per weight, especially from 1 to 25 % by weight, phosphate powder (based on the total weight of phosphate powder and already baked material).

6. Formation material according to one of the preceding claims, **characterised in that** the matrix of the augmentation material is tightly sintered to microporosity.

7. Formation material according to one of the preceding claims, **characterised by** a microporosity of 20 % by volume or more, preferably from 20 to 40 % by volume, and especially 30 % by volume or more, based on the overall porosity (consisting of micro- and macro-porosity).

8. Formation material according to one of the preceding claims, obtainable by providing the compressed blank with tubular pores with the aid of a compression mould of optionally more than one part.

9. Formation material according to one of the preceding claims, obtainable by providing the baked blank with tubular pores by means of milling or drilling.

10. Formation material according to one of the preceding claims, **characterised in that** the formation material is in block form, with 2- or 3-dimensionally oriented macroscopic tubular pores passing through each block, which are in each case arranged perpendicular to the block surface or to an imaginary plane laid through the block or against the block and form an interconnecting system of tubular pores.

11. Formation material according to claim 10, **characterised in that** a block intended for implantation, together with its tubular pores, can be so orientated for implantation or on processing prior to implantation that at least one direction of orientation of the tubular pores corresponds to a biomechanically or biofunctionally intended direction of growth.

12. Formation material according to one of the preceding claims, **characterised by** tubular pores that have radii in the region of from 100 to 2000µm and especially from 500 to 2000µm.

13. Formation material according to one of the preceding claims, **characterised in that** the formation material, present in block form, is penetrated by the tubular pores spaced apart at a defined spacing with respect to one another, especially at a spacing that corresponds to a wall thickness of not more than from 1500 to 4000µm and especially from 2000 to 3000µm.

14. Formation material according to one of the preceding claims, **characterised by** an overall porosity (consisting of micro- and macro-porosity) of more than 50 % by volume.

15. Formation material according to one of the preceding claims, **characterised by** a macroporosity of from 25 to 50 % by volume, and especially from 30 to 40 % by volume, based on the overall porosity (consisting of micro- and macro-porosity).

16. Formation material according to one of the preceding claims, **characterised in that** the block form is a simple geometric shape, especially that of a cube, cuboid, taper, cone or disc.

17. Formation material according to one of the preceding claims, **characterised in that** it is a semi-finished product, especially for subsequent mechanical processing, preferably for individual adaptation in the case of a bone defect in mouth or jaw medicine, orthopaedic surgery or trauma surgery.

18. Formation material according to one of the claims 11 to 17, **characterised in that** the material is compressed, especially baked or sintered, only to a degree such that it can be processed using tools available to the practitioner, especially using a rasp, file, scalpel or a dentist's instrument.

19. Individual prosthesis, which can be produced by bringing a formation material according to one of claims 11 to 17 into the form of the individual prosthesis with the aid of a medical CAD/CAM method.

## Revendications

1. Matériau résorbable pour le remplacement ou la reconstitution de tissus osseux (matériau augment) à base de β-phosphate tricalcique (β-TCP) poreux, susceptible d'être préparé par
(a) calcination d'une poudre de phosphate ayant une composition chimique telle que le résidu de calcination donne théoriquement du phosphate tricalcique chimiquement pur, et
(b) réalisation de pores tubulaires dans les pièces brutes calcinées ainsi obtenues,
**caractérisé par le fait que** l'on procède à au moins deux, en particulier à au moins trois opérations de calcination du β-phosphate tricalcique (β-TCP) et que l'on prévient la formation des phases proches du β-TCP qui sont thermodynamiquement stables,
(i) en réduisant en poudre le produit de pré-synthèse obtenu dans l'étape (a),
(ii) en soumettant éventuellement le produit de pré-synthèse réduit en poudre, mélangé à de la poudre de phosphate de l'étape (a), à une calcination et en réduisant en poudre le matériau ainsi formé, et en répétant éventuellement l'étape (ii) une ou plusieurs fois,
(iii) en comprimant le produit pulvérisé formé dans l'étape (i) ou dans l'étape (ii) avec de la poudre de phosphate de l'étape (a), pour en faire des pièces brutes, et en soumettant les pièces brutes formées à une cuisson céramique finale, et
(iv) en soumettant à l'étape (b) les pièces brutes, comprimées ou cuites, constituées pour au moins 99,5 % de β-phosphate tricalcique pur (β-TCP).

2. Matériau de reconstitution selon la revendication 1, **caractérisé par le fait que** la pureté chimique et cristalline, la structure, la microporosité et la macroporosité du matériau augment permettent une intégration et une résorption rapides dans l'os, qui s'effectuent par voie biochimique et sans réaction de rejet de corps étranger.

3. Matériau de reconstitution selon l'une des revendications précédentes, que l'on peut fabriquer:
(i) en partant d'un produit de pré-synthèse que l'on peut obtenir par calcination d'une poudre de phosphate ayant une composition chimique telle que le résidu de calcination donne théoriquement du phosphate tricalcique chimiquement pur en tant que produit de pré-synthèse, et en réduisant en poudre ce produit de pré-synthèse,
(ii) en calcinant éventuellement le produit de pré-synthèse réduit en poudre, mélangé à de la poudre de phosphate de l'étape (i), et en réduisant en poudre le matériau ainsi obtenu, et en répétant éventuellement une ou plusieurs fois l'étape (ii),
(iii) en comprimant le produit pulvérisé formé dans l'étape (i) ou dans l'étape (ii), mélangé à de la poudre de phosphate de l'étape (i), pour en faire des pièces brutes, et en soumettant les pièces brutes formées à une cuisson céramique finale, et
(iv) en pratiquant des pores tubulaires dans les pièces brutes comprimées ou cuites.

4. Matériau de reconstitution selon la revendication 1 ou 3, que l'on peut obtenir en calcinant à une température inférieure à 1200 °C dans le domaine d'existence du β-phosphate tricalcique (β-TCP).

5. Matériau de reconstitution selon l'une quelconque des revendications 1, 3 ou 5, que l'on peut obtenir en utilisant dans l'étape (ii) et/ou dans l'étape (iii) de 1 à 50 % en poids, en particulier de 1 à 25 % en poids de poudre de phosphate (rapporté au poids total de poudre de phosphate et de matériau déjà calciné).

6. Matériau de reconstitution selon l'une quelconque des revendications précédentes, **caractérisé par le fait que** la matrice du matériau augment est compactée par frittage jusqu'à microporosité.

7. Matériau de reconstitution selon l'une quelconque des revendications précédentes, **caractérisé par** une microporosité supérieure ou égale à 20 % en volume, de préférence comprise entre 20 % et 40 % en volume, et en particulier supérieure ou égale à 30 % en volume, rapporté à la porosité totale (microporosité et macroporosité).

8. Matériau de reconstitution selon l'une quelconque des revendications précédentes, que l'on peut obtenir en pratiquant des pores tubulaires dans une pièce brute comprimée, à l'aide d'un moule de compression comportant éventuellement plusieurs parties.

9. Matériau de reconstitution selon l'une quelconque des revendications précédentes, que l'on peut obtenir en pratiquant des pores tubulaires dans une pièce brute cuite, par fraisage ou forage.

10. Matériau de reconstitution selon l'une quelconque des revendications précédentes, **caractérisé par le fait qu'**il se présente sous forme de blocs, chaque bloc étant traversé de pores tubulaires macroscopiques à orientation bi- ou tridimensionnelle, qui sont chacun perpendiculaires à la surface du bloc ou à un plan imaginaire traversant le bloc ou tangent à celui-ci, et qui forment un système de pores tubulaires communicants.

11. Matériau de reconstitution selon la revendication 10, **caractérisé par le fait qu'**un bloc à implanter, comportant des pores tubulaires, peut être orienté pour une implantation ou lors d'une opération précédant l'implantation, de manière à ce qu'au moins une direction d'orientation des pores tubulaires corresponde à une direction prévisible de croissance biomécanique ou biofonctionnelle.

12. Matériau de reconstitution selon l'une quelconque des revendications précédentes, **caractérisé par le fait que** les pores tubulaires ont un rayon compris de 100 à 2000 µm, et en particulier, de 500 à 2000 µm.

13. Matériau de reconstitution selon l'une quelconque des revendications précédentes, **caractérisé par le fait que** les pores tubulaires traversent le bloc de matériau de reconstitution à une distance réciproque définie, en particulier à une distance correspondant à une épaisseur de paroi valant au plus 1500 à 4000 µm, et en particulier, 2000 à 3000 µm.

14. Matériau de reconstitution selon l'une quelconque des revendications précédentes, **caractérisé par le fait que** sa porosité totale (microporosité et macroporosité) est supérieure à 50 % en volume.

15. Matériau de reconstitution selon l'une quelconque des revendications précédentes, **caractérisé par le fait que** sa macroporosité représente de 25 à 50 % en volume, et en particulier de 30 à 40 % en volume, de la porosité totale (microporosité et macroporosité).

16. Matériau de reconstitution selon l'une quelconque des revendications précédentes, **caractérisé par le fait que** le bloc a une forme géométrique simple, en particulier une forme de cube, de parallélépipède rectangle, de cône, de cylindre ou de disque.

17. Matériau de reconstitution selon l'une quelconque des revendications précédentes, **caractérisé par le fait qu'**il s'agit d'un produit semi-fini, en particulier pour post-usinage mécanique, de préférence pour adaptation individuelle à un défaut osseux dans le domaine de la médecine de la bouche ou de la mâchoire, de la chirurgie orthopédique ou de la chirurgie post-traumatique.

18. Matériau de reconstitution selon l'une quelconque des revendications 11 à 17, **caractérisé par le fait que** le matériau n'a été compacté, en particulier par cuisson ou par frittage, qu'à un point tel qu'il peut être usiné au moyen d'outils dont peut disposer un médecin, par exemple à l'aide d'une râpe, d'une lime, d'un scalpel ou d'un instrument de dentisterie.

19. Prothèse individuelle que l'on peut préparer en donnant à un matériau de reconstitution selon l'une quelconque des revendications 11 à 17 la forme d'une prothèse individuelle, à l'aide d'un système de CFAO pour la médecine.
